# EUROPÄISCHE PATENTSCHRIFT

(11) **EP 4 333 914 B1**
(45) Veröffentlichungstag und Bekanntmachung des Hinweises auf die Patenterteilung: **01.04.2026**
(21) Anmeldenummer: 22726066.8
(22) Anmeldetag: 27.04.2022
(51) Int. Cl.: A61L 27/14, A61L 27/22, A61L 27/38

(54) **VERFAHREN ZUR HERSTELLUNG VON VASKULARISIERTEM BIOLOGISCHEM GEWEBE**
METHOD FOR PRODUCING VASCULARIZED BIOLOGICAL TISSUE
PROCÉDÉ DE FABRICATION DE TISSU BIOLOGIQUE VASCULARISÉ

(30) Priorität: 07.05.2021 DE 102021111956
(43) Veröffentlichungstag der Anmeldung: 13.03.2024
(73) Patentinhaber: Fraunhofer-Gesellschaft zur Förderung der angewandten Forschung e.V., 80686 München (DE); Universität des Saarlandes, 66123 Saarbrücken (DE)
(72) Erfinder: ZIMMERMANN, Heiko, 66280 Sulzbach (DE); GEPP, Michael, 97082 Würzburg (DE); FISCHER, Benjamin, 80686 München (DE)
(74) Vertreter: v. Bezold & Partner Patentanwälte - PartG mbB
(86) Internationale Anmeldenummer: PCT/EP2022/061249
(87) Internationale Veröffentlichungsnummer: WO 2022/233680

(56) Entgegenhaltungen:
- EP-A1- 3 418 375
- WO-A1-2019/226710
- WO-A1-2020/174468

## Beschreibung

Die Erfindung betrifft ein Verfahren zur Herstellung von biologischem Gewebe, insbesondere von Gewebe aus Herzmuskelzellen, Leberzellen, Nierenzellen, Nervenzellen und/oder Pankreaszellen. Anwendungen der Erfindung bestehen z. B. in der Biomedizin, insbesondere beim so genannten Tissue engineering.

Es ist allgemein bekannt, biologisches Gewebe, umfassend eine Vielzahl differenzierter biologischer Zellen mit einer gemeinsamen Funktion, für Forschungszwecke oder für Implantationszwecke mit biotechnologischen Verfahren nachzubilden. Beispielsweise können Herzmuskelzellen durch Differenzierung aus pluripotenten Stammzellen gewonnen und durch Kultivierung in einem Inkubator vermehrt werden. Herkömmliche Nachbildungen von biologischem Gewebe (Gewebeprodukte), die eine Anhäufung von differenzierten Zellen umfassen, haben jedoch die folgenden Nachteile, wodurch sich Beschränkungen bei der Anwendung von Gewebeprodukten und deren Kosten ergeben.

Erstens weist natürliches biologisches Gewebe nicht nur die differenzierten Zellen, sondern auch Blutgefäße und eine extrazelluläre Matrix auf. Erst im Verbund mit den weiteren Gewebebestandteilen liefert die Anhäufung von differenzierten Zellen eine Anpassung an die Eigenschaften natürlichen biologischen Gewebes. Nachbildungen von biologischem Gewebe können daher bisher nur beschränkt die Funktionen des natürlichen Gewebes erfüllen. Des Weiteren ist die Größe von herkömmlichen Nachbildungen von biologischem Gewebe begrenzt, da Zellen im Inneren einer Zellkultur ggf. nicht optimal mit Nährstoffen und Sauerstoff versorgt werden können und absterben. Insbesondere bei Kultivierung im adhärenten Zustand haben herkömmliche Nachbildungen von biologischem Gewebe häufig eine Schichtform, die nicht optimal an die natürliche Raumform eines Gewebes angepasst ist. Bei der Implantation herkömmlicher Gewebeprodukte in einen biologischen Organismus kann es zu unerwünschten Immunreaktionen oder sogar Abstoßungsreaktionen kommen, da die Gewebeprodukte aufgrund ihrer von den natürlichen Geweben abweichenden Zusammensetzung als Fremdmaterialien erkannt werden.

WO 2019/226710 A1 offenbart ein Verfahren zur Herstellung von vaskularisiertem biologischen Gewebe, umfassend die Schritte: Herstellung einer Netzwerkstruktur aus einer Vielzahl von miteinander verbundenen Filamenten eines Stützpolymers; Beschichtung der Netzwerkstruktur mit einem Proteinmaterial; Besiedlung der beschichteten Netzwerkstruktur mit Endothelzellen und mit Gewebe-bildenden biologischen Zellen; und Auflösung der Filamente der Netzwerkstruktur, so dass das vaskularisierte Gewebe gebildet wird. Die Herstellung der Netzwerkstruktur umfasst eine Deposition der Filamente auf einem mit einem degradierbaren Matrixmaterial beschichteten Trägersubstrat und eine nachfolgende Ablösung der Netzwerkstruktur vom Trägersubstrat.

Die Aufgabe der Erfindung ist es, ein verbessertes Verfahren zur Herstellung von biologischem Gewebe bereitzustellen, mit dem Beschränkungen herkömmlicher Techniken vermieden werden.

Das Verfahren zur Herstellung von biologischem Gewebe soll sich insbesondere durch die Schaffung eines Gewebes auszeichnen, dessen Eigenschaften besser an Eigenschaften eines natürlichen Gewebes angepasst sind, das mit größeren Dimensionen herstellbar ist, das mit einer frei wählbaren Raumform herstellbar ist, das sich durch verminderte Immunreaktionen auszeichnet, das kostengünstiger herstellbar ist, und/oder das neue oder erweiterte Anwendungen von Gewebeprodukten ermöglicht.

Diese Aufgabe wird durch ein Verfahren zur Herstellung von biologischem Gewebe gelöst, das die Merkmale von Anspruch 1 aufweist. Bevorzugte Ausführungsformen und Anwendungen der Erfindung ergeben sich aus den abhängigen Ansprüchen.

Gemäß einem allgemeinen Gesichtspunkt der Erfindung wird die obige Aufgabe durch ein Verfahren zur Herstellung von vaskularisiertem biologischem Gewebe gemäß Anspruch 1 gelöst. Das Verfahren umfasst die Schritte Herstellung einer Netzwerkstruktur aus einer Vielzahl von miteinander verbundenen Filamenten eines Stützpolymers, Beschichtung der Netzwerkstruktur mit einem Proteinmaterial, Besiedlung der beschichteten Netzwerkstruktur mit Endothelzellen und mit Gewebe-bildenden biologischen Zellen, und Auflösung der Filamenten der Netzwerkstruktur, so dass das vaskularisierte Gewebe gebildet wird.

Erfindungsgemäß wird mit der beschichteten Netzwerkstruktur ein ein- oder mehrlagiges Volumen-Substrat bereitgestellt, das durch die Filamente des Stützpolymers gebildet wird. Die Filamente, die auch als Fäden oder Fasern oder längliche Polymerabschnitte bezeichnet werden können, bilden z. B. eine ungeordnete oder eine regelmäßige Verteilung im Raum, in der die Filamente punktuell oder abschnittsweise in gegenseitigem Kontakt oder voneinander beabstandet sind. Dadurch wird eine innere Oberfläche der Netzwerkstruktur geschaffen, welche die Beschichtung der Netzwerkstruktur mit dem Proteinmaterial und die nachfolgende Besiedlung mit den Gefäßzellen (z. B. Endothelzellen) und den Gewebe-bildenden biologischen Zellen, optional unter Zusatz von extrazellulärer Matrix (ECM), ermöglicht. Die Filamente sind vorzugsweise aus einem Polysaccharid, wie z. B. Alginat oder Gelatine, hergestellt. Die Beschichtung der Netzwerkstruktur umfasst insbesondere eine Beschichtung der Filamente im Inneren der Netzwerkstruktur mit dem Proteinmaterial. Das Proteinmaterial fördert vorteilhafterweise ein adhärentes Wachstum der Zellen auf den Filamenten.

Zur Besiedlung der beschichteten Netzwerkstruktur mit den Endothelzellen und den Gewebe-bildenden biologischen Zellen erfolgt eine Inkubation der Netzwerkstruktur mit einer Zell-Suspension, so dass die Zellen auf den Filamenten adhärent anhaften, und eine Kultivierung der angehafteten Zellen, vorzugsweise mit einer Zufuhr von Kultivierungsmedium (Nährmedium). Vorzugsweise werden zuerst die Endothelzellen und nach Bildung einer Lage (Mehrfachlage, Monolage oder sub-Monolage) von Endothelzellen nachfolgend die Gewebe-bildenden biologischen Zellen in die Netzwerkstruktur eingeführt.

Die Endothelzellen sind differenzierte Zellen, welche im natürlichen Gewebe die Innenseite der Blutgefäße bilden. Es werden vorzugsweise Endothelzellen des Organismus, z. B. eines Säugetieres, insbesondere Menschen, verwendet, dessen Gewebe nachgebildet werden soll. Die Gewebe-bildenden biologischen Zellen sind differenzierte Zellen von mindestens einem Zelltyp, aus denen das nachzubildenden Gewebe aufgebaut werden soll. Die Differenzierung der Gewebe-bildenden biologischen Zellen erfolgt vorzugsweise vor der Besiedlung der Netzwerkstruktur.

Die Auflösung der Netzwerkstruktur ist nach der Besiedlung der Netzwerkstruktur mit den Zellen vorgesehen. Die Auflösung der Netzwerkstruktur umfasst vorzugsweise eine chemische und/oder thermische Umwandlung der Filamente in einen flüssigen Zustand und ein Ausspülen des umgewandelten Materials aus dem angesiedelten Zellverbund. Durch die Auflösung der Netzwerkstruktur bleiben im Verbund der Zellen miteinander verbundene Hohlräume zurück, deren innere Oberflächen durch die Endothelzellen gebildet werden. Die Erfinder haben festgestellt, dass diese Hohlräume geometrisch wie Blutgefäße im natürlichen Gewebe verteilt sind und entsprechend Blutgefäße im vaskularisierten Gewebe bilden, welches vorzugsweise das fertige Gewebeprodukt darstellt.

Die Auflösung der Netzwerkstruktur erfolgt vorzugsweise, nachdem die Besiedlung vollständig abgeschlossen und das innere Volumen der Netzwerkstruktur mit den Zellen dicht gefüllt ist. Alternativ kann die Auflösung bereits bei teilweiser Füllung des inneren Volumens der Netzwerkstruktur vorgesehen sein, so dass das Wachstum weiterer Gefäße erleichtert wird.

Vorteilhafterweise werden mit dem erfindungsgemäß hergestellten vaskularisierten Gewebe die folgenden Vorteile erzielt.

Die Eigenschaften des vaskularisierten Gewebes sind an Eigenschaften des entsprechenden natürlichen Gewebes besser angepasst als dies bei herkömmlichen Gewebeprodukten der Fall ist. Das vaskularisierte Gewebe kann sogar von natürlichem Gewebe ununterscheidbar sein. Entsprechend ist auch eine Verringerung von Immunreaktionen bei Implantation des Gewebeprodukts in einen lebenden Organismus möglich.

Des Weiteren werden die Größenbeschränkungen herkömmlicher Gewebeprodukte überwunden, indem die differenzierten Zellen im Geweben über die Gefäße im vaskularisierten Gewebe versorgt werden können. Das vaskularisierte Gewebe ist nicht auf eine Schichtform beschränkt, sondern kann mit einer frei wählbaren Raumform herstellbar ist, die an die Form des natürlichen Gewebes oder eines gewünschten Implantats angepasst ist.

Das erfindungsgemäße Verfahren wirkt sich positiv auf die Kosten des vaskularisierten Gewebes aus, das mit größeren Volumen und größeren Mengen mit erhöhter Effektivität herstellbar ist als herkömmliche Gewebeprodukte.

Das erfindungsgemäß hergestellte vaskularisierte Gewebe bietet auch neue Anwendungen von Gewebeprodukten insbesondere in der Forschung und in der Implantationsmedizin, z. B. bei der Schaffung von Modellgeweben, die sich von natürlichem Gewebe nicht oder nur vernachlässigbar unterscheiden.

Die Erfindung ist mit verschiedenen Gewebe-bildenden Zellen anwendbar. Gemäß einer bevorzugten Anwendung der Erfindung umfassen die Gewebe-bildenden Zellen Herzmuskelzellen und das vaskularisierte Gewebe ein Herzmuskelgewebeprodukt. Alternativ umfassen die Gewebe-bildenden Zellen Leberzellen, Nierenzellen, Nervenzellen oder Pankreaszellen, wobei das vaskularisierte Gewebe entsprechend ein Lebergewebeprodukt, Nierengewebeprodukt, Nervengewebeprodukt oder Pankreasgewebeprodukt ist. Das vaskularisierte Gewebe kann alternativ mehrere Zelltypen enthalten, wie z. B. Herzmuskel- und Nervenzellen oder andere Zellen in Kombination mit Nervenzellen.

Vorteilhafterweise sind verschiedene Ausführungsformen der Erfindung verfügbar, bei denen die Herstellung der Netzwerkstruktur auf einem festen Trägersubstrat oder ohne eine Bindung an ein festes Trägersubstrat erfolgt.

Gemäß einer bevorzugten Ausführungsform der Erfindung umfasst die Herstellung der Netzwerkstruktur eine Deposition der Filamente auf einem mit einem degradierbaren Matrixmaterial, wie z. B. einem Polysaccharid, insbesondere Dextran, beschichteten Trägersubstrat und eine spätere Ablösung der Netzwerkstruktur vom Trägersubstrat. Vorteilhafterweise wird die Netzwerkstruktur durch das Trägersubstrat unterstützt.

Die Deposition der Filamente umfasst eine Anordnung einer Vielzahl von vorab hergestellten, z. B. extrudierten, Filamenten des Stützpolymers und/oder eine Abscheidung des Stützpolymers mit einem 3D-Abscheideverfahren derart, dass die Filamente auf dem Trägersubstrat aufgebaut werden. Ein 3D-Abscheideverfahren umfasst z. B. ein 3D-Druckverfahren, insbesondere ein 3D-Gefrier-Drucken, bei dem die Filamente des Stützpolymers im gefrorenen Zustand gebildet werden.

Das Trägersubstrat wird in einem Vorbereitungsschritt unmittelbar vor der Deposition der Filamente mit dem Matrixmaterial beschichtet werden. Vorzugsweise wird als Trägersubstrat der Boden eines Gefäßes verwendet, das bei den nachfolgenden Schritten der Beschichtung der Filamente und der Besiedlung mit Zellen Lösungen oder Suspensionen der jeweils zugeführten Komponenten aufnimmt und einen Inkubator bildet. Das Gefäß ist z. B. ein Reaktionsgefäß einer Reaktionsplatte, wie z. B. einer Mikrotiterplatte.

Das Matrixmaterial bildet eine Isolationsschicht. Die Ablösung der Netzwerkstruktur vom Trägersubstrat umfasst eine Auflösung des Matrixmaterials. Das Matrixmaterial unterscheidet sich vorzugsweise in seiner chemischen Löslichkeit von der Löslichkeit der Filamente. Besonders bevorzugt sind die Filamente und das Matrixmaterial aus verschiedenen Polysacchariden hergestellt. Vorteilhafterwiese bleiben damit bei der Ablösung der Netzwerkstruktur vom Trägersubstrat die Filamente zunächst erhalten.

Bei dieser Ausführungsform der Erfindung ist somit vorzugsweise eine zweistufige Auflösung des Matrixmaterials und des Stützpolymers, besonders bevorzugt aus Polysacchariden, vorgesehen, bei der zuerst eine Ablösung der Netzwerkstruktur von Trägersubstrat und anschließend nach der Besiedlung mit den Zellen eine Auflösung der Netzwerkstruktur erfolgt.

Gemäß der Erfindung ist eine Bildung eines Netzwerkstruktur-Zell-Verbundes durch die Beschichtung der Netzwerkstruktur mit dem Proteinmaterial und die Besiedlung der beschichteten Netzwerkstruktur mit den Endothelzellen und mit den Gewebe-bildenden biologischen Zellen vorgesehen, bevor die Netzwerkstruktur vom Trägersubstrat abgelöst wird, wobei Seitenabschnitte der Filamente im Netzwerkstruktur-Zell-Verbund (d. h. eine seitliche Oberfläche des Netzwerkstruktur-Zell-Verbundes) das Trägersubstrat berühren, wobei anschließend die Schritte Ablösung des Netzwerkstruktur-Zell-Verbundes vom Trägersubstrat, Faltung des Netzwerkstruktur-Zell-Verbundes zu einer Mehrfachschicht derart, dass sich die Seitenabschnitte der Filamente im Netzwerkstruktur-Zell-Verbund mindestens teilweise berühren, und Fixierung des gefalteten Netzwerkstruktur-Zell-Verbundes mit der nachfolgenden Auflösung der Netzwerkstruktur vorgesehen sind.

Indem die Beschichtung der Netzwerkstruktur mit dem Proteinmaterial und die Besiedlung der beschichteten Netzwerkstruktur ausgeführt werden, bevor die Netzwerkstruktur vom Trägersubstrat abgelöst wird, erfüllt das Trägersubstrat seine Stützfunktion vorteilhafterweise bis zu der Anordnung der Zellen im Verbund, wobei die Anordnung der Filamente und damit der nachfolgend gebildeten Blutgefäße erhalten bleibt. Durch die Faltung des Netzwerkstruktur-Zell-Verbundes zu einer Mehrfachschicht wird vorteilhafterweise die Bildung einer Raumform des Gewebeprodukts vereinfacht.

Vorteilhafterweise sind verschiedene Varianten der Faltung des Netzwerkstruktur-Zell-Verbundes verfügbar, die einzeln oder in Kombination ausgeführt werden können. Gemäß einer ersten Variante ist ein Aufhängen des Netzwerkstruktur-Zell-Verbundes über einem länglichen Halteelement, wie z. B. einem Haltefaden oder einem Haltestab derart vorgesehen, dass Oberflächen des Netzwerkstruktur-Zell-Verbundes, an denen die Seitenabschnitte der Filamente freiliegen, sich berühren. Der Netzwerkstruktur-Zell-Verbund wird am länglichen Halteelement umgeschlagen, so dass sich zwei durch das längliche Halteelement getrennte Abschnitte einer Oberfläche des Netzwerkstruktur-Zell-Verbundes aneinanderlegen. Vorteilhafterweise haften die Abschnitte der Oberfläche aneinander, so dass ein geschlossener Zellverbund entsteht, von dem das längliche Halteelement leicht getrennt werden kann.

Gemäß einer zweiten Variante ("Origami"-Variante) ist eine Ablage des Netzwerkstruktur-Zell-Verbundes auf einem Faltungssubstrat derart, dass die Seitenabschnitte der Filamente freiliegen, und eine Deformation des Faltungssubstrats derart vorgesehen, dass Oberflächen des Netzwerkstruktur-Zell-Verbundes, an denen die Seitenabschnitte der Filamente freiliegen, sich berühren. Vorteilhafterweise liefert die Deformation des Faltungssubstrats eine Kraftwirkung, unter der sich die Abschnitte einer seitlichen Oberfläche des Netzwerkstruktur-Zell-Verbundes berühren, wodurch die adhärente Verbindung der Teile des Netzwerkstruktur-Zell-Verbundes unterstützt wird.

Gemäß einer dritten Variante ist eine Ablage des Netzwerkstruktur-Zell-Verbundes auf einem Faltungswerkzeug derart, dass die Seitenabschnitte der Filamente freiliegen, und eine Betätigung des Faltungswerkzeugs derart vorgesehen, dass Oberflächen des Netzwerkstruktur-Zell-Verbundes, an denen die Seitenabschnitte der Filamente freiliegen, sich berühren. Auch in diesem Fall wird mit dem Faltungswerkzeug vorteilhafterweise eine Kraft ausgeübt, unter der sich der Zellverbund im gefalteten Zustand stabilisiert.

Besonders bevorzugt ist die Netzwerkstruktur in Bezug auf eine vorbestimmte Referenzebene senkrecht zur Ausdehnung der Netzwerkstruktur spiegelsymmetrisch geformt, wobei die Faltung des Netzwerkstruktur-Zell-Verbundes entlang der Referenzlinie erfolgt. Entsprechend berühren sich nach der Faltung Filamentabschnitte mit gleichen Formen, so dass nach der Auflösung der Filamente größere Gefäßdurchmesser erzielt werden.

Gemäß einer alternativen Ausführungsform der Erfindung ist die Beschichtung der Netzwerkstruktur mit dem Proteinmaterial und die Besiedlung der beschichteten Netzwerkstruktur mit den Endothelzellen und mit den Gewebe-bildenden biologischen Zellen vorgesehen, nachdem die Netzwerkstruktur vom Trägersubstrat abgelöst worden ist. In diesem Fall können sich Vorteile durch eine vereinfachte Zufuhr des Proteinmaterials, der Endothelzellen und der Gewebe-bildenden biologischen Zellen von mehreren Seiten in die Netzwerkstruktur.

Gemäß einer weiteren bevorzugten Ausführungsform der Erfindung umfasst die Herstellung der Netzwerkstruktur vorzugsweise eine 3D-Abscheidung der Filamente ohne eine Bindung an ein festes Trägersubstrat. Die 3D-Abscheidung kann z. B. in einer hochviskosen Trägerflüssigkeit oder einem weichen Polymer, insbesondere einem Substratblock, oder auf einer Lage von vorgefertigten Filamenten erfolgen.

Der Begriff "3D-Abscheidung" bezieht sich allgemein auf Verfahren, mit denen ein dreidimensionales Gebilde aus den Filamenten durch Aufbau des Stützpolymers mit oder ohne Substratbindung hergestellt wird. Die Anwendung der 3D-Abscheidung hat den besonderen Vorteil, dass die Verteilung der Filamente und damit der Blutgefäße im vaskularisierten Gewebe durch die ortsaufgelöste Steuerung der Abscheidung des Stützpolymers vorgegeben werden kann.

Vorteilhafterweise kann die 3D-Abscheidung der Filamente ein 3D-Gefrier-Drucken des Stützpolymers umfassen, bei dem eine Lösung des Stützpolymers bei einer Temperatur unterhalb des Gefrierpunkts des Stützpolymers schichtweise aufgebaut und vernetzt wird, so dass die Anordnung der Filamente mit einem inneren Volumen gebildet wird.

Alternativ kann die 3D-Abscheidung der Filamente ein Extrudieren des Stützpolymers mit einer Kanüleneinrichtung in ein Trägermaterial umfassen. Besonders bevorzugt umfasst die Kanüleneinrichtung eine Koaxialkanüle, mit der gleichzeitig das Stützpolymer und die Endothelzellen in das Trägermaterial eingeführt werden.

Vorzugsweise umfasst das Stützpolymer Alginat, wobei die Auflösung des Stützpolymers unter Verwendung von Alginatlyase erfolgt. Die Verwendung von Alginat hat besondere Vorteile aufgrund der Biokompatibilität von Alginat und dessen Eignung für eine rückstandfreie Auflösung zur Bildung der Gefäße im Zellverbund. Alternativ oder zusätzlich sind andere Uronsäure-basierte Polysaccharide, wie z. B. Galacturonsäure, oder Protein-basierte Stützpolymere verwendbar, wie z. B. Gelatine. Die Auflösung des Stützpolymers erfolgt vorzugsweise unter Verwendung von Alginatlyase, Dextranase und/oder Pectinase. Des Weiteren kann ein Komplexbildner, wie z.B. EDTA (Ethylendiamintetraessigsäure) für das Auflösen der Stützstruktur verwendet werden.

Ein besonderer Vorteil der Erfindung besteht darin, dass das vaskularisierte Gewebe in frei wählbaren Größen herstellbar ist. Vorteilhafterweise kann eine Verbindung von mindestens zwei Lagen vaskularisierten Gewebes zu einem Gewebeblock vorgesehen sein.

Vorteilhafterweise erlaubt das erfindungsgemäße Verfahren eine Modifizierung des Zellverbundes zur Herstellung des vaskularisierten Gewebes, beispielsweise um dessen Versorgung zu verbessern. Vorzugsweise ist gemäß einer Ausführungsform der Erfindung eine Einbettung von mindestens einer Perfusionsleitung in den Zellverbund zur Herstellung des vaskularisierten Gewebes, in das vaskularisierte Gewebes und/oder in den Gewebeblock vorgesehen, wobei die Perfusionsleitung aus einem lösbaren Material hergestellt und zur Zufuhr eines Kultivierungsmediums angeordnet ist. Die Perfusionsleitung erlaubt z. B. in einer ersten Kultivierungsphase des vaskularisierten Gewebes die verstärkte Zufuhr des Kultivierungsmediums, um die Zellvermehrung im vaskularisierten Gewebe zu unterstützen, wobei die Perfusionsleitung in einer späteren Kultivierungsphase des vaskularisierten Gewebes oder bei dessen Anwendung, wenn die inneren Gefäße ausreichend stark ausgebildet sind, aufgelöst werden kann.

Weitere Einzelheiten und Vorteile der Erfindung werden im Folgenden unter Bezug auf die beigefügten Zeichnungen beschrieben. Die Zeichnungen zeigen schematisch in:
- Figuren 1 bis 5:: Merkmale des Verfahrens zur Herstellung von vaskularisiertem Gewebe gemäß einer Ausführungsform der Erfindung,
- Figuren 6 bis 9:: Merkmale des Verfahrens zur Herstellung von vaskularisiertem Gewebe gemäß weiteren Ausführungsformen der Erfindung,
- Figur 10:: die Herstellung von vaskularisiertem Gewebe mit eingebetteten Perfusionsleitungen, und
- Figur 11:: die Herstellung von vaskularisiertem Gewebe in Gestalt von einem Gewebeblock.

Merkmale von Ausführungsformen der Erfindung werden im Folgenden unter beispielhaftem Bezug auf die Herstellung von vaskularisiertem Herzmuskelzellengewebe beschrieben. Es wird betont, dass die Umsetzung der Erfindung in der Praxis nicht auf die Anwendung mit Herzmuskelzellen beschränkt, sondern auch mit anderen Zelltypen möglich ist, wie z. B. Leberzellen, Nierenzellen, Nervenzellen und/oder Pankreaszellen. Einzelheiten der konkret verwendeten Zelltypen von Endothelzellen und Herzmuskelzellen, deren Bereitstellung, z. B. durch Differenzierung aus pluripotenten Stammzellen, und Kultivierung werden nicht beschrieben, da diese an sich aus dem Stand der Technik bekannt sind.

Die Figuren illustrieren Ausführungsformen der Erfindung anhand von vergrößerten Schnittansichten, die jeweils einen einlagigen oder zweilagigen Ausschnitt einer Netzwerkstruktur oder eines vaskularisierten Gewebes im Bereich weniger Filamente oder weniger Gefäße zeigen. In der Praxis wird die Erfindung mit ausgedehnten Netzwerkstrukturen realisiert, die erheblich mehr Filamente bzw. Gefäße und/oder mehr Lagen oder eine Raumform der Netzwerkstruktur umfassen können.

Die Herstellung von vaskularisiertem biologischen Gewebe erfolgt unter Verwendung von Techniken, die von biotechnologischen Aufgaben im Labor oder eine industriellen Produktion an sich bekannt sind, wie z. B. die Verwendung von Inkubatoren mit Einrichtungen zur Zuführung von Lösungen und/oder Suspensionen.

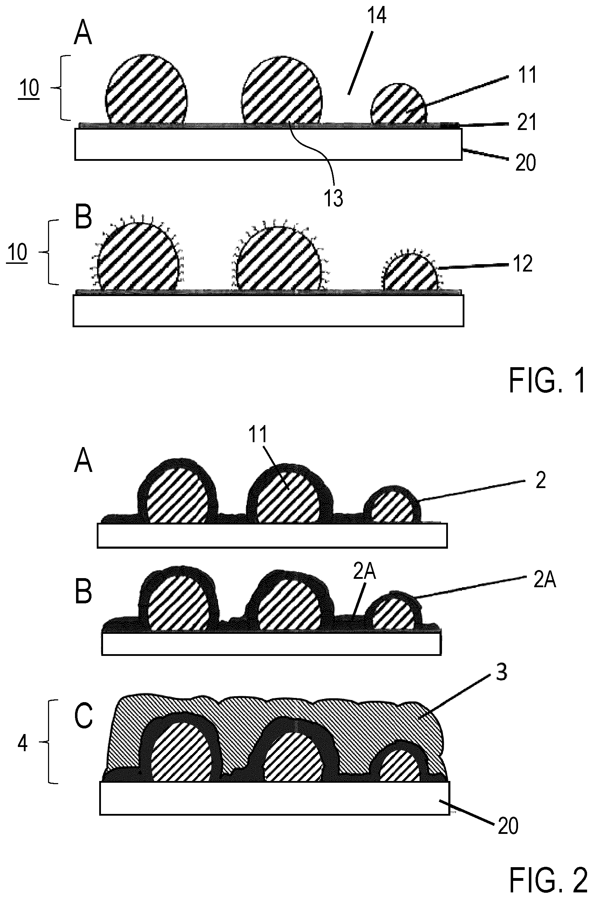

Die Figuren 1 bis 5 illustrieren eine erste Ausführungsform des erfindungsgemäßen Verfahrens zur Herstellung von vaskularisiertem Gewebe, bei dem die Herstellung einer Netzwerkstruktur und deren Besiedlung mit Endothelzellen und Herzmuskelzellen auf einem festen Trägersubstrat erfolgen.

Die Figuren 1A und 1B zeigen die Herstellung der Netzwerkstruktur 10 aus einer Vielzahl von miteinander verbundenen Filamenten 11 eines Stützpolymers, wie z. B. Alginat, und die Beschichtung der Netzwerkstruktur 10 mit einem Proteinmaterial 12, wie z. B. Fibronektin oder Laminin.

Das feste Trägersubstrat 20 mit einer vorzugsweise ebenen Oberfläche wird z. B. aus PMMA oder Glas hergestellt. Das Trägersubstrat 20 wird beispielsweise durch den Boden eines Napfes einer Mikrotiterplatte (Mikrowell) gebildet und mit einem Matrixmaterial 21, wie z. B. Dextran, beschichtet. Die Dicke der vorzugsweise geschlossenen Schicht des Matrixmaterials 21 beträgt z. B. 10 µm.

Die Filamente 11, die in den Figuren in Schnittansicht gezeigt sind und sich außerhalb der Zeichenebene berühren und/oder kreuzen können, werden gemäß Figur 1A z. B. als vorgefertigtes Netz ausgefällter Alginat-Filamente oder durch 3D-Abscheidung auf dem Trägersubstrat 10 ungeordnet oder mit einer regelmäßigen Verteilung angeordnet. Seitenabschnitte 13 der Filamente 11 berühren das beschichtete Trägersubstrat 20 und Abstände 14 verbleiben zwischen den Filamenten 11. Die Durchmesser der Alginat-Filamente 11 sind vorzugsweise im Bereich von 50 µm bis 500 µm oder auch über 500 µm gewählt. Die Abstände 14 haben eine Größe im Bereich von 100 µm bis 500 µm.

Das Proteinmaterial 12 wird gemäß Figur 1B z. B. aus einer Umgebungslösung über dem beschichteten Trägersubstrat 20 oder durch eine gezielte Tropfendeposition auf den Filamenten 11 und den freiliegenden Bereichen des Matrixmaterials 21 abgeschieden.

Anschließend erfolgt gemäß Figur 2 die Bildung eines Netzwerkstruktur-Zell-Verbundes 4. Hierzu wird zunächst ein erster Typ von Endothelzellen 2 auf den Filamenten 11 und den freiliegenden Bereichen des Matrixmaterials 21 adhärent haftend angesiedelt (siehe Figur 2A). Die Zufuhr der Zellen erfolgt aus einer Suspension, die das beschichtete Trägersubstrat 20 und die Filamente 11 bedeckt. Vorzugsweise wird eine geschlossene Schicht (Mono- oder Mehrfachlage) der Endothelzellen 2 gebildet. Optional wird ergänzend ein zweiter Typ von Zellen 2A, wie z. B. glatte Muskelzellen, auf den Filamenten 11 und/oder den Bereichen zwischen den Filamenten 11 angesiedelt (siehe Figur 2B). Durch die Verwendung von zwei oder mehr Typen von Endothelzellen kann vorteilhafterweise die Zusammensetzung von Gefäßwänden ähnlich wie in natürlichen Geweben gebildet werden. Optional kann eine Kultivierung der Endothelzellen 2, 2A im adhärenten Zustand auf dem Trägersubstrat 20 unter Zufuhr eines Kultivierungsmediums vorgesehen sein, wobei sich die Endothelzellen 2, 2A vermehren.

Auf den Endothelzellen 2, 2A werden nachfolgend die Gewebe-bildenden Zellen 3, wie z. B. Herzmuskelzellen, angeordnet (siehe Figur 2C). Die Zufuhr der Herzmuskelzellen erfolgt wieder aus einer Suspension, die das beschichtete Trägersubstrat 20 und die mit den Endothelzellen 2, 2A versehenen Filamente 11 bedeckt. Die Gewebe-bildenden Zellen 3 bilden eine geschlossene Schicht, die sich über die Filamente 11 und die Bereiche zwischen den Filamenten 11 erstreckt. Die Gesamtdicke des damit hergestellten Netzwerkstruktur-Zell-Verbundes 4 beträgt z. B. 5 mm.

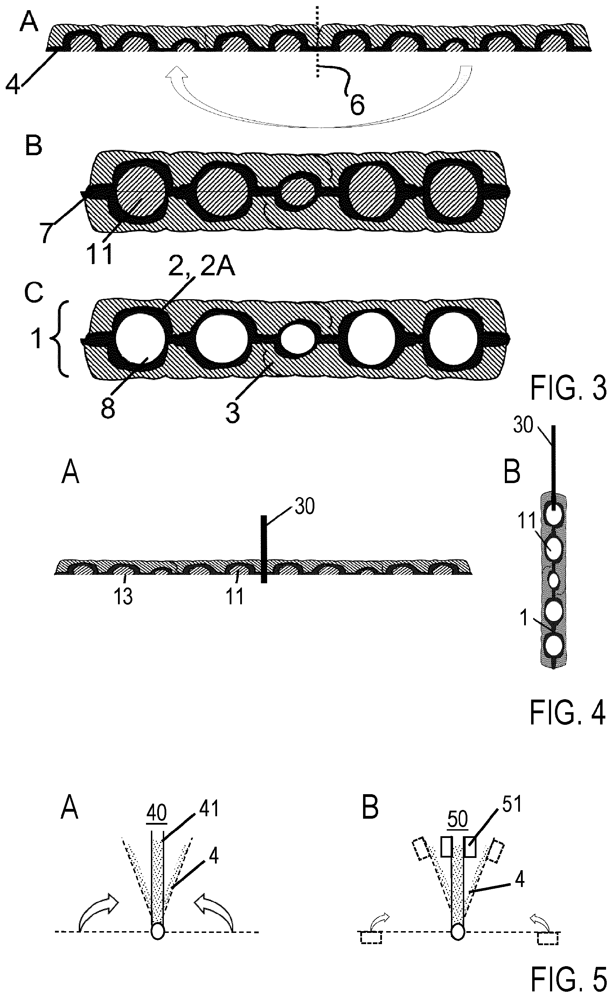

Der Netzwerkstruktur-Zell-Verbund 4 wird vom Trägersubstrat 20 abgelöst. Hierzu wird das Matrixmaterial 21 durch Zufuhr eines Lösungsmittels, welches das Matrixmaterial 21, jedoch nicht das Stützpolymer der Filamente auflöst, wie z. B. Dextranase, aufgelöst, so dass sich der Netzwerkstruktur-Zell-Verbund 4 vom Trägersubstrat 20 trennt (siehe Figur 3A).

Vorzugsweise ist die Netzwerkstruktur 10 in Bezug auf eine vorbestimmte Referenzebene 6 senkrecht zur Ausdehnung der Netzwerkstruktur 10 spiegelsymmetrisch geformt, wie in Figur 3A schematisch gezeigt ist. Durch eine Faltung des Netzwerkstruktur-Zell-Verbundes 4 an der Referenzebene (siehe Pfeil in Figur 3A) passen die Filamente mit gleichen Positionen relativ zur Faltungslinie 7 und gleichen Größen zueinander (siehe Figur 3B). Aus den Filamenten 11, die zunächst aufgrund des Kontakts mit dem Trägersubstrat 20 seitlich abgeplattet waren, entstehen allseits abgerundete Filamente, deren Querschnittform an die Querschnittsform der zu bildenden Gefäße angepasst ist. Im gefalteten Zustand werden sich berührende Oberflächen des Netzwerkstruktur-Zell-Verbundes 4 miteinander verbunden, wodurch der Netzwerkstruktur-Zell-Verbund 4 fixiert wird.

Die Symmetrie in Bezug auf die Referenzebene 6 ist kein zwingendes Merkmal der Erfindung. Auch bei nicht-symmetrischen Verteilungen ergibt eine Faltung des Netzwerkstruktur-Zell-Verbundes 4 durch Selbstorganisationsprozesse eine Verteilung von sich berührenden Filamenten 11, die für die nachfolgende Vaskularisierung vorgesehen sind.

Anschließend erfolgt die Auflösung der Netzwerkstruktur 10, so dass das vaskularisierte Gewebe 1 gebildet wird (siehe Figur 3C). Die Auflösung der Netzwerkstruktur 10 erfolgt z. B. durch Zufuhr von Alginatlyase. Im Ergebnis setzt sich das vaskularisierte Gewebe 1 aus den Gewebe-bildenden Zellen 3 zusammen, in deren Verbund nach Auflösung der Filamente Hohlräume verbleiben. Die Hohlräume sind miteinander verbunden, so dass sie im vaskularisierten Gewebe 1 Gefäße 8 bilden. Innenwände der Gefäße 8 werden durch die Endothelzellen 2, 2A gebildet.

Die Figuren 4 und 5 zeigen Varianten der Faltung des Netzwerkstruktur-Zell-Verbundes 4 durch Aufhängung an einem länglichen Halteelement 30 (Figur 4), unter Verwendung eines Faltungssubstrats 40 (Figur 5A) oder unter Verwendung eines Faltungswerkzeugs 50 (Figur 5B).

Das längliche Halteelement 30 ist z. B. ein Haltefaden, über den der Netzwerkstruktur-Zell-Verbund 4 entlang der Schnittlinie der Referenzebene 6 mit dem Netzwerkstruktur-Zell-Verbund 4 gelegt wird (siehe Figur 4A). Oberflächen des Netzwerkstruktur-Zell-Verbundes 4, an denen die Seitenabschnitte 13 der Filamente 11 freiliegen, schwenken beidseitig des Halteelements 30 unter der Wirkung der Gravitation, bis sie sich berühren und durch die Bildung von Zell-Zell-Kontakten verbinden (siehe Figur 4B). Anschließend können das Halteelement 30 entfernt und die Filamente 11 aufgelöst werden, um das vaskularisierte Gewebe 1 zu bilden.

Das Faltungssubstrat 40 ist ein faltbares Trägerelement mit zwei ebenen Flügeln, das zwischen einem ausgebreiteten, ebenen Zustand (in Figur 5A gestrichelt gezeigt) in einen zusammengeklappten Zustand (in Figur 5A mit durchgezogenen Linien gezeigt) verschwenkbar ist. Das Faltungssubstrat 40 besteht zum Beispiel aus einem elastisch federnden, bistabilen Material, das durch innere mechanische Spannungen zwischen den beiden Zuständen verschwenkbar ist. Man kann mit dem Faltungssubstrat 40 zwei Teile eines Netzwerkstruktur-Zell-Verbundes 4 flächig aneinander legen und kontaktieren. Der Netzwerkstruktur-Zell-Verbund 4 wird auf das Faltungssubstrat 40 im ausgebreiteten Zustand aufgelegt, wobei die Schnittlinie der Referenzebene 6 mit dem Netzwerkstruktur-Zell-Verbund 4 (siehe Figur 3) mit dem Verlauf der Schwenkachse zwischen den beiden Flügeln des Faltungssubstrats 40 zusammenfällt. Durch den Übergang in den zusammengeklappten Zustand (siehe Pfeile) erfolgt eine Deformation des Faltungssubstrats 40 derart, dass Oberflächen des Netzwerkstruktur-Zell-Verbundes 4, an denen die Seitenabschnitte der Filamente freiliegen, sich berühren und durch die Bildung von Zell-Zell-Kontakten miteinander verbinden. Anschließend können das Faltungssubstrat 40 entfernt und die Filamente aufgelöst werden, um das vaskularisierte Gewebe zu bilden.

Das Faltungswerkzeug 50 gemäß Figur 5B hat eine ähnliche Funktion wie das Faltungssubstrat 40, indem es zwischen einem ausgebreiteten, ebenen Zustand (in Figur 5B gestrichelt gezeigt) in einen zusammengeklappten Zustand (in Figur 5B mit durchgezogenen Linien gezeigt) verschwenkbar ist. Abweichend von der inneren Spannung des Faltungssubstrats 40 wird das Faltungswerkzeug 50 an mindestens einem Aktor 51 betätigt, um den Netzwerkstruktur-Zell-Verbund 4 zu falten.

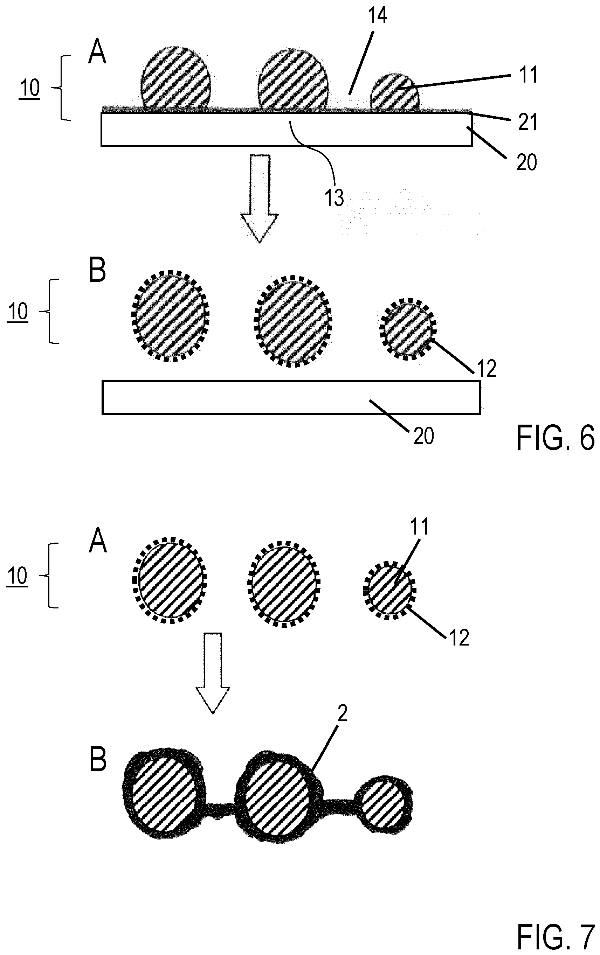

Die Figuren 6 bis 8 illustrieren eine zweite Ausführungsform des erfindungsgemäßen Verfahrens zur Herstellung von vaskularisiertem Gewebe, bei dem nur die Herstellung der Netzwerkstruktur auf einem festen Trägersubstrat erfolgt. Anschließend wird die Netzwerkstruktur vom Trägersubstrat abgelöst, mit einem Proteinmaterial beschichtet und mit Endothelzellen und Herzmuskelzellen besiedelt.

Figur 6A zeigt die Herstellung der Netzwerkstruktur 10 aus einer Vielzahl von miteinander verbundenen Filamenten 11 eines Stützpolymers, wie z. B. Alginat, mit gegenseitigen Abständen 14 auf dem mit einem Matrixmaterial 21 beschichteten Trägersubstrat 20, wie in Figur 1A. Die Netzwerkstruktur 10 wird anschließend vom Trägersubstrat 20 abgelöst und allseits mit dem Proteinmaterial 12 beschichtet (Figur 6B und 7A).

Die vom Trägersubstrat 20 abgelöste und mit dem Proteinmaterial 12 beschichtete Netzwerkstruktur 10 wird mit Endothelzellen 2 besiedelt (Figur 7B). Hierzu wird die Netzwerkstruktur 10 in einem Inkubator mit einer Suspension der Endothelzellen 2 inkubiert.

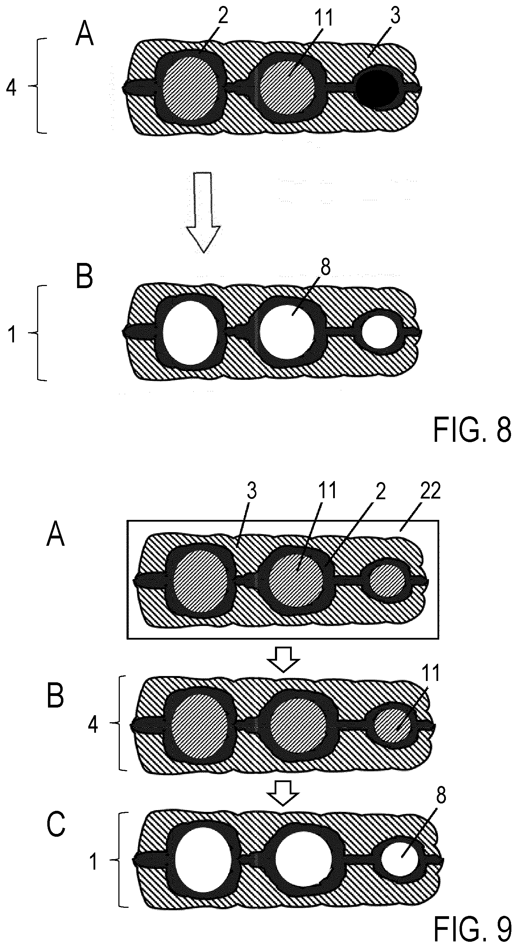

Durch eine Inkubation mit einer Herzmuskelzellen-Suspension werden Herzmuskelzellen 3 auf den Endothelzellen 2 angesiedelt, die auf den Filamenten 11 und zwischen diesen angeordnet sind, wodurch der Netzwerkstruktur-Zell-Verbund 4 gebildet wird (Figur 8A). Anschließend wird das Stützpolymer der Filamente 11 aufgelöst, wodurch im Innern des Netzwerkstruktur-Zell-Verbundes 4 und damit das vaskularisierte Gewebe 1 gebildet wird.

Figur 9 illustrieren eine dritte Ausführungsform des erfindungsgemäßen Verfahrens zur Herstellung von vaskularisiertem Gewebe, bei dem die Herstellung der Netzwerkstruktur und die Besiedlung mit Endothelzellen und Herzmuskelzellen ohne Bindung an ein festes Trägersubstrat, sondern in einem Substratblock, z. B. aus einem weichen, nicht vollständig vernetzten Alginat erfolgen. Das Alginat ist so weich gebildet, dass Zellen im Alginat sich ausrichten können und zugleich von ihrer Umgebung getrennt werden. Anschließend wird die Netzwerkstruktur aus dem Substratblock herausgelöst und das Stützpolymer der Filamente aufgelöst.

Gemäß Figur 9A werden die Filamente 11 des Stützpolymers, wie z. B. Alginat oder Galacturonsäure, mittels einer Kanüle eingebettet. Vorzugsweise wird eine Koaxialkanüle verwendet, mit deren innerem Kanal das Stützpolymer, insbesondere schwach vernetztes Alginat und optional extrazelluläre Matrix (ECM) für eine Polarisierung der Zellen, und mit deren äußerem Kanal die Endothelzellen 2 gemeinsam mit ECM und optional auch einem Zusatz von Ethylendiamintetraessigsäure (EDTA) zugeführt werden. Die Koaxialkanüle wird gemäß einem vorgegebenen Programm im Substratblock 22 platziert und unter Abgabe des Stützpolymers und der Endothelzellen 2 mit den Zusätzen bewegt, z. B. entlang vorgegebener Bahnen zurückgezogen. Anschließend werden mit einer Kanüle Herzmuskelzellen 3 mit Zusätzen aus ECM und optional auch EDTA zugeführt und auf den Endothelzellen 2 platziert, so dass im Substratblock 22 der Netzwerkstruktur-Zell-Verbund 4 gebildet wird.

Im weiteren Verfahren wird der Netzwerkstruktur-Zell-Verbund 4 vom Substratblock 22 getrennt, indem der Substratblock 22 mit Alginatlyase aufgelöst wird (Figur 9B). Anschließend werden die Filamente 11 z. B. mit Pectinase aufgelöst, wodurch die freien Gefäße 8 des vaskularisierten Gewebes 1 gebildet werden (Figur 9C).

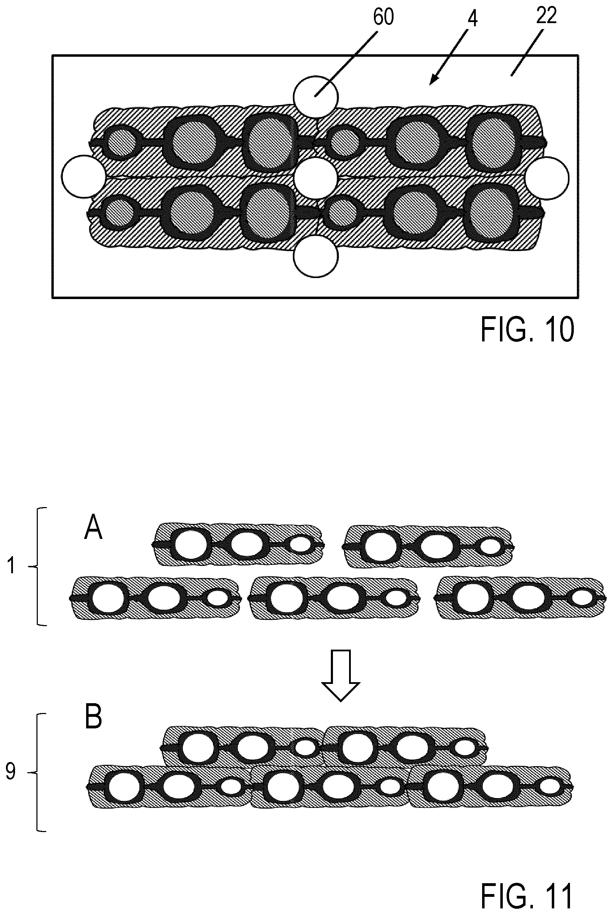

Figur 10 illustriert eine Abwandlung des Verfahrens gemäß Figur 9, wobei zusätzlich im Netzwerkstruktur-Zell-Verbund 4 Perfusionsleitungen 60 zur zusätzlichen Versorgung der Zellen mit Kultivierungsmedium eingebettet werden, insbesondere während der Netzwerkstruktur-Zell-Verbund 4 im Substratblock 22 kultiviert wird. Die Perfusionsleitungen 60 dienen insbesondere der Versorgung während der Reifung und Organisation des Netzwerkstruktur-Zell-Verbundes 4, und sie sind vorzugsweise aus einem degradierbaren Material, wie z. B. Dextran hergestellt. Beispielsweise können Dialyseschläuche als Perfusionsleitungen 60 verwendet werden. Wenn der Netzwerkstruktur-Zell-Verbund 4 vom Substratblock 22 getrennt wird, können die Perfusionsleitungen 60 z. B. mit Dextranase aufgelöst werden.

Mehrere Abschnitte des vaskularisierten Gewebes 1, das insbesondere mit einem Verfahren gemäß einer der beschriebenen Ausführungsformen hergestellt ist, können zu einem Gewebeblock 9 verbunden werden, wie schematisch in Figur 11 gezeigt ist. Die Verbindung erfolgt durch gegenseitige Berührung unter Zusatz von Genipin.

Die in der vorstehenden Beschreibung, den Zeichnungen und den Ansprüchen offenbarten Merkmale der Erfindung können sowohl einzeln als auch in Kombination oder Unterkombination für die Verwirklichung der Erfindung in ihren verschiedenen Ausgestaltungen von Bedeutung sein.

## Patentansprüche

1. Verfahren zur Herstellung von vaskularisiertem biologischen Gewebe (1), umfassend die Schritte
- Herstellung einer Netzwerkstruktur (10) aus einer Vielzahl von miteinander verbundenen Filamenten (11) eines Stützpolymers, wobei die Herstellung der Netzwerkstruktur (10) eine Deposition der Filamente (11) auf einem mit einem degradierbaren Matrixmaterial (21) beschichteten Trägersubstrat (20) und eine nachfolgende Ablösung der Netzwerkstruktur (10) vom Trägersubstrat (20) umfasst,
- Beschichtung der Netzwerkstruktur (10) mit einem Proteinmaterial (12),
- Besiedlung der beschichteten Netzwerkstruktur (10) mit Endothelzellen (2, 2A) und mit Gewebe-bildenden biologischen Zellen (3), und
- Auflösung der Filamente (11) der Netzwerkstruktur (10), so dass das vaskularisierte Gewebe (1) gebildet wird,
wobei das Verfahren die Schritte aufweist:
- Bildung eines Netzwerkstruktur-Zell-Verbundes (4) durch die Beschichtung der Netzwerkstruktur (10) mit dem Proteinmaterial (12) und die Besiedlung der beschichteten Netzwerkstruktur (10) mit den Endothelzellen (2, 2A) und mit den Gewebe-bildenden biologischen Zellen (3), bevor die Netzwerkstruktur (10) vom Trägersubstrat (20) abgelöst wird, wobei Seitenabschnitte (13) der Filamente (11) im Netzwerkstruktur-Zell-Verbund (4) das Trägersubstrat (20) berühren,
- Ablösung des Netzwerkstruktur-Zell-Verbundes (4) vom Trägersubstrat (20),
- Faltung des Netzwerkstruktur-Zell-Verbundes (4) zu einer Mehrfachschicht (5) derart, dass sich die Seitenabschnitte (13) der Filamente (11) im Netzwerkstruktur-Zell-Verbund (4) mindestens teilweise berühren, und
- Fixierung des gefalteten Netzwerkstruktur-Zell-Verbundes (4) mit der nachfolgenden Auflösung der Netzwerkstruktur (10).

2. Verfahren gemäß Anspruch 1, wobei die Faltung des Netzwerkstruktur-Zell-Verbundes (4) umfasst
- ein Aufhängen des Netzwerkstruktur-Zell-Verbundes (4) über einem länglichen Halteelement (30), so dass Oberflächen des Netzwerkstruktur-Zell-Verbundes (4), an denen die Seitenabschnitte (13) der Filamente (11) freiliegen, sich berühren,
- eine Ablage des Netzwerkstruktur-Zell-Verbundes (4) auf einem Faltungssubstrat (40) derart, dass die Seitenabschnitte (13) der Filamente (11) freiliegen, und eine Deformation des Faltungssubstrats derart, dass Oberflächen des Netzwerkstruktur-Zell-Verbundes (4), an denen die Seitenabschnitte (13) der Filamente (11) freiliegen, sich berühren, oder
- eine Ablage des Netzwerkstruktur-Zell-Verbundes (4) auf einem Faltungswerkzeug (50) derart, dass die Seitenabschnitte (13) der Filamente (11) freiliegen, und eine Betätigung des Faltungswerkzeugs derart, dass Oberflächen des Netzwerkstruktur-Zell-Verbundes (4), an denen die Seitenabschnitte (13) der Filamente (11) freiliegen, sich berühren.

3. Verfahren gemäß Anspruch 1 oder 2, bei dem
- die Netzwerkstruktur (10) in Bezug auf eine vorbestimmte Referenzebene (6) senkrecht zur Ausdehnung der Netzwerkstruktur (10) spiegelsymmetrisch geformt ist, und
- die Faltung des Netzwerkstruktur-Zell-Verbundes (4) entlang der Referenzebene (6) erfolgt.

4. Verfahren gemäß Anspruch 1, bei dem
- die Herstellung der Netzwerkstruktur (10) eine 3D-Abscheidung der Filamente (11) umfasst, derart, dass die Filamente (11) auf dem festen Trägersubstrat (20) aufgebaut werden.

5. Verfahren gemäß Anspruch 4, bei dem
- die 3D-Abscheidung der Filamente (11) ein 3D-Gefrier-Drucken des Stützpolymers umfasst.

6. Verfahren gemäß einem der vorhergehenden Ansprüche, mit mindestens einem der Merkmale
- das Stützpolymer umfasst Alginat, ein anderes Uronsäure-basierte Polysaccharid, insbesondere Galacturonsäure, und/oder ein Protein-basiertes Stützpolymere, insbesondere Gelatine, und
- die Auflösung des Stützpolymers erfolgt unter Verwendung von Alginatlyase, Dextranase, Pectinase und/oder einem Komplexbildner, insbesondere EDTA (Ethylendiamintetraessigsäure).

7. Verfahren gemäß einem der vorhergehenden Ansprüche, ferner umfassend
- Verbinden von mindestens zwei Lagen vaskularisierten Gewebes (1) zu einem Gewebeblock (9).

8. Verfahren gemäß einem der vorhergehenden Ansprüche, ferner umfassend
- eine Einbettung von mindestens einer Perfusionsleitung (60) in das vaskularisierte Gewebe (1) oder den Gewebeblock (9), wobei die Perfusionsleitung (60) aus einem lösbaren Material hergestellt und zur Zufuhr eines Kultivierungsmediums in das vaskularisierte Gewebe (1) oder den Gewebeblock (9) angeordnet ist.

9. Verfahren gemäß einem der vorhergehenden Ansprüche, bei dem
- die Gewebe-bildenden biologischen Zellen (3) Herzmuskelzellen, Leberzellen, Nierenzellen, Nervenzellen und/oder Pankreaszellen umfassen.

## Claims

1. A method for manufacturing vascularized biological tissue (1), comprising the steps of
- manufacturing a network structure (10) from a plurality of interconnected filaments (11) of a supporting polymer, wherein the manufacturing of the network structure (10) comprises a deposition of the filaments (11) on a carrier substrate (20) coated with a degradable matrix material (21) and a subsequent detachment of the network structure (10) from the carrier substrate (20),
- coating the network structure (10) with a protein material (12),
- colonization of the coated network structure (10) with endothelial cells (2, 2A) and with tissue-forming biological cells (3), and
- dissolving the filaments (11) of the network structure (10) so that the vascularized tissue (1) is formed,
wherein the method comprises the steps of:
- forming a network structure-cell-composite (4) by coating the network structure (10) with the protein material (12) and colonizing the coated network structure (10) with the endothelial cells (2, 2A) and with the tissue-forming biological cells (3) before the network structure (10) is detached from the carrier substrate (20), wherein side portions (13) of the filaments (11) in the network structure-cell-composite (4) contact the carrier substrate (20),
- detaching the network structure-cell-composite (4) from the carrier substrate (20),
- folding the network structure-cell-composite (4) to form a multilayer (5) in such a way that the side sections (13) of the filaments (11) in the network structure-cell-composite (4) at least partially touch each other, and
- fixing the folded network structure-cell-composite (4) with the subsequent dissolution of the network structure (10).

2. The method according to claim 1, wherein the folding of the network structure-cell-composite (4) comprises
- suspending the network structure-cell-composite (4) over an elongate holding element (30) so that surfaces of the network structure-cell-composite (4) where the side portions (13) of the filaments (11) are exposed are in contact,
- depositing the network structure-cell-composite (4) on a folding substrate (40) such that the side portions (13) of the filaments (11) are exposed, and deforming the folding substrate such that surfaces of the network structure-cell-composite (4) where the side portions (13) of the filaments (11) are exposed are in contact, or
- depositing the network structure-cell-composite (4) on a folding tool (50) such that the side portions (13) of the filaments (11) are exposed, and actuating the folding tool such that surfaces of the network structure-cell-composite (4) at which the side portions (13) of the filaments (11) are exposed are in contact.

3. The method according to claim 1 or 2, wherein
- the network structure (10) is shaped with mirror symmetry with respect to a predetermined reference plane (6) perpendicular to the extension of the network structure (10), and
- the network structure-cell-composite (4) is folded along the reference plane (6).

4. The method according to claim 1, wherein
- the manufacturing of the network structure (10) comprises a 3D deposition of the filaments (11) in such a way that the filaments (11) are built up on the solid carrier substrate (20).

5. The method according to claim 4, wherein
- the 3D deposition of the filaments (11) comprises 3D freeze printing the supporting polymer.

6. The method according to any one of the preceding claims, having at least one of the features
- the supporting polymer comprises alginate, another uronic acid-based polysaccharide, in particular galacturonic acid, and/or a protein-based supporting polymer, in particular gelatin, and
- the supporting polymer is dissolved using alginate lyase, dextranase, pectinase and/or a complexing agent, in particular EDTA (ethylenediaminetetraacetic acid).

7. The method according to any one of the preceding claims, further comprising
- joining at least two layers of vascularized tissue (1) to form a tissue block (9).

8. The method according to any one of the preceding claims, further comprising
- an embedding of at least one perfusion pipe (60) in the vascularized tissue (1) or the tissue block (9), wherein the perfusion pipe (60) is made of a dissolvable material and is arranged for supplying a cultivation medium into the vascularized tissue (1) or the tissue block (9).

9. The method according to any one of the preceding claims, wherein
- the tissue-forming biological cells (3) comprise heart muscle cells, liver cells, kidney cells, nerve cells and/or pancreatic cells.

## Revendications

1. Procédé de fabrication de tissu biologique vascularisé (1), comprenant les étapes
- fabrication d'une structure de réseau (10) à partir d'une pluralité de filaments (11) interconnectés d'un polymère de support, dans lequel la fabrication de la structure de réseau (10) comprend un dépôt des filaments (11) sur un substrat de support (20) revêtu d'un matériau matriciel (21) dégradable et un détachement qui suit de la structure de réseau (10) du substrat de support (20),
- revêtement de la structure de réseau (10) avec un matériau protéique (12),
- colonisation de la structure de réseau (10) revêtue avec des cellules endothéliales (2, 2A) et avec des cellules biologiques formant des tissus (3), et
- dissolution des filaments (11) de la structure de réseau (10) de manière à former le tissu vascularisé (1),
dans lequel le procédé présente les étapes :
- formation d'un assemblage de structure de réseau-cellule (4) par le revêtement de la structure en réseau (10) avec le matériau protéique (12) et colonisation de la structure en réseau (10) revêtue par les cellules endothéliales (2, 2A) et les cellules biologiques formant des tissus (3), avant que la structure de réseau (10) ne soit détachée du substrat de support (20), dans lequel des sections latérales (13) des filaments (11) dans l'assemblage structure de réseau-cellule (4) touchent le substrat de support (20),
- détachement de l'assemblage structure de réseau-cellule (4) du substrat de support (20),
- pliage de l'assemblage de structure de réseau-cellule (4) en une couche multiple (5) de telle manière que les sections latérales (13) des filaments (11) dans l'assemblage structure de réseau-cellule (4) se touchent au moins en partie, et
- fixation de l'assemblage structure de réseau-cellule (4) plié avec la dissolution qui suit de la structure de réseau (10).

2. Procédé selon la revendication 1, dans lequel le pliage de l'assemblage structure de réseau-cellule (4) comprend
- une suspension de l'assemblage structure de réseau-cellule (4) au-dessus d'un élément de maintien allongé (30), de telle sorte que des surfaces de l'assemblage structure de réseau-cellule (4), sur lesquelles les sections latérales (13) des filaments (11) sont exposées, se touchent,
- un dépôt de l'assemblage structure de réseau - cellule (4) sur un substrat de pliage (40) de telle manière que les sections latérales (13) des filaments (11) sont exposées, et une déformation du substrat de pliage de telle manière que des surfaces de l'assemblage structure de réseau-cellule (4), sur lesquelles les sections latérales (13) des filaments (11) sont exposées, se touchent, ou
- un dépôt de l'assemblage structure de réseau-cellule (4) sur un outil de pliage (50) de telle manière que les sections latérales (13) des filaments (11) sont exposées, et un actionnement de l'outil de pliage de telle manière que des surfaces de l'assemblage structure de réseau-cellule (4), sur lesquelles les sections latérales (13) des filaments (11) sont exposées, se touchent.

3. Procédé selon la revendication 1 ou 2, dans lequel
- la structure de réseau (10) est formée de manière symétrique en miroir par rapport à un plan de référence (6) prédéterminé perpendiculairement à l'extension de la structure de réseau (10), et
- le pliage de l'assemblage structure de réseau-cellule (4) est effectué le long du plan de référence (6) .

4. Procédé selon la revendication 1, dans lequel
- la fabrication de la structure de réseau (10) comprend un dépôt 3D des filaments (11) de telle manière que les filaments (11) sont montés sur le substrat de support fixe (20).

5. Procédé selon la revendication 4, dans lequel
- la séparation 3D des filaments (11) comprend une impression 3D par congélation du polymère de support.

6. Procédé selon l'une quelconque des revendications précédentes, avec au moins une des caractéristiques
- le polymère de support comprend de l'alginate, un autre polysaccharide à base d'acide uronique, en particulier de l'acide galacturonique, et/ou un polymère de support à base de protéines, en particulier de la gélatine, et
- la dissolution du polymère de support est effectuée en utilisant de l'alginate lyase, de la dextranase, de la pectinase et/ou un agent complexant, en particulier de l'EDTA (acide éthylènediaminetétraacétique).

7. Procédé selon l'une quelconque des revendications précédentes, comprenant en outre
- liaison d'au moins deux couches de tissu vascularisé (1) en un bloc de tissu (9).

8. Procédé selon l'une quelconque des revendications précédentes, comprenant en outre
- une insertion d'au moins une ligne de perfusion (60) dans le tissu vascularisé (1) ou le bloc de tissu (9), dans lequel la ligne de perfusion (60) est fabriquée à partir d'un matériau soluble et est disposée dans le tissu vascularisé (1) ou le bloc de tissu (9) pour l'apport d'un milieu de culture.

9. Procédé selon l'une quelconque des revendications précédentes, dans lequel
- les cellules biologiques formant des tissus (3) comprennent des cellules musculaires cardiaques, des cellules hépatiques, des cellules rénales, des cellules nerveuses et/ou des cellules pancréatiques.
